# EUROPEAN PATENT SPECIFICATION

(11) **EP 0 852 938 B1**
(45) Date of publication and mention of the grant of the patent: **16.03.2005**
(21) Application number: 98100166.2
(22) Date of filing: 07.01.1998
(51) Int. Cl.: A61F 13/15

(54) **Absorbent products having conforming means**
Absorbierende Produkte mit Anpassungshilfen
Articles absorbants avec moyen de conformation

(30) Priority: 08.01.1997 US 779549
(43) Date of publication of application: 15.07.1998
(73) Proprietor: McNeil-PPC, Inc., Skillman, NJ 08558-9418 (US)
(72) Inventor: Ulman, John, Woodbridge, NJ 07095 (US); Glasgow, Tara, Yardley, PA 19067 (US); Rosenfeld, Leonard, East Windsor, NJ 08520 (US); Ehrhard, Joseph A., Flemington, NJ 08822 (US)
(74) Representative: Groening, Hans Wilhelm, Dipl.-Ing.

(56) References cited:
- EP-A- 0 136 524
- EP-A- 0 621 082
- WO-A-91/14415
- US-A- 3 575 174

## Description

The invention regards an absorbent product according the preamble part of claim 1.

Such absorbent product relates to protective absorbent products used by women for feminine hygiene, and more particularly to improved sanitary protection products such as sanitary napkins, panty liners and incontinence products that, in use, fit closely and conform to the body, resist transverse bunching and prevent leakage of fluid from the edges.

Protective absorbent products are commonly used by women for feminine hygiene is to absorb body fluids, such as menstrual fluids, intermenstrual fluids and urine. It is important that these products prevent such fluids from escaping the confines of the absorbent materials and edges of such products and thereby staining the wearer's undergarments and outer clothing. Current product designs attempt to provide coverage of the body without being bunched, folded and otherwise having their absorbent regions reduced in size and removed from contact with the body; fit close to the body to prevent fluid from running along the body before it is absorbed; quickly absorb and retain the fluids and do not permit them to escape the confines of the porous surfaces of the product, or their external edges, onto the wearer's clothing; and by being comfortable and not irritating to the wearer. Examples of such products are sanitary napkins that additionally have: flaps or wings attached to the edges of the napkins, such as are described by McNair in US 4,285,343 and by Matttingly in US 4,608,047, or attached to a fixation point on the absorbent element of the napkin inwardly of the longitudinal edges of the napkin, as described by McCoy in US 4,900,320; cuffs; interlabial protrusions; and extra absorbent materials, layers and structures strategically placed throughout the absorbent body of the napkin.

Attempts have been to prevent a napkin from bending and bunching, especially in the rearward portion of the napkin, by reducing the size of the rearward portion or by providing a preformed arcuate profile in the rearward portion of the napkin, the drawback being that such means further limit the absorbent area of the rearward portion. Several inventions, that attempt to prevent a napkin from bending and bunching, describe the use of predominantly centrally located channels, pleats or slits that generally follow the longitudinal dimension of the napkin, some of which also reduce the width of the product or promote reduction of its width in use; and none of which help the ends of the product conform and adapt to the surface of the body in use. Thus, Strongson in US 2, 331, 355 describes a catamenial pad having a central gathered, extra thickness portion of reduced width which is attained by gathering the pad about a pleat below the central portion; or by splitting the absorbent material in the central portion longitudinally, as well as optionally outward from the longitudinal split, and gathering the absorbent material upward between the slits. Romans-Hess et al in US 4,655,759 describe a sanitary napkin that has embossed channels, located adjacent the longitudinal edges, that in use are said to permit the sides of the napkin to fold upward to form an occlusive container. However, the sides of such napkins, in use, either stay flat or fold downward, thereby reducing the strike zone available to accept body fluids. Glassman in US 4,758,240 describes a menstrual or incontinent pad that embodies a longitudinal centrally located interlabial-entering ridge bearing a deep channel, the pad having on either side of the ridge a multitude of spaced apart channels that increase the lateral compressibility of the pad, without it folding downward, when the pad is subjected to lateral compression in use. Pigneul in US 4,790,838 describes a sanitary napkin comprising a fluid absorbent pad component having: biconcave longitudinal sides that define a biconcave central area of reduced width that is contained between a fluid permeable cover and an impermeable barrier; and a wide wing area extending beyond the pad component in the biconcave region, the wide wing area consisting only of the cover and barrier sealed to each other in arcuate pleats that are concentric with the biconcave sides of the pad. A further feature of the device includes one or more arcuate grooves in the mass of the pad component, that are preferably concentric with the concavities of the central area. Glaug et al in US 5,399,175 describe an absorbent structure provided with two or more longitudinal chambers of absorbent material that are separated by repellent walls, that direct fluid along the longitudinal axis of the structure.

Several patents provide transverse channels alone or in combination with longitudinal channels. None of these, for reasons to be discussed, have the effect of significantly helping the ends of the product conform and adapt to the surface of the body in use. Thus, Mogor in US 3,575,174 describes the use of deep embossed channels impressed through the cover and core of a napkin, the channels being positioned near the lateral and end edges, on the body facing surface of the napkin, as well as at the rearward end of the napkin on the garment facing surface of the napkin. The channels on the body facing surface, although providing resistance to bending and bunching, also prevent the front and rear regions of the napkin from adapting and conforming to the mons pubis and to the posterior perineum. Molee et al, in US 4,773,905 and in US 4,936,839, describe winged napkins having a fluid retarding means, for example embossed channels, disposed transversely across a central absorbent element to retard the transmission of fluid from the central portion of the absorbent element to its transverse end portions. The napkin may also include fluid barrier seals, between the absorbent element and the wings, as well as fluid repellent means around the napkin's periphery. Finally, Lindquist in WO 90/05514 describes an absorbent article having an absorbent body that includes a plurality of mutually sequential channel forming impressions, that have both transverse and longitudinal extending parts that are located at a distance from the periphery of the absorbent body; and where preferably that distance is such that the distance between mutually adjacent channels is smaller than the distance of the channels to the nearest edge of the absorbent body. The objectives of this last cited invention are to provide a preferred path of wicking of fluid from channel to channel instead of to the edges; to provide lateral stability by virtue of the lateral extending parts of the channels; and to provide flexibility along the longitudinal axis of the article at hinge sections between sequential channels. However, to provide a preferred path for wicking fluid, such sequential channel impressions must be at a distance from the periphery and quite close to each other; and will not adequately; resist sideways compression; prevent fluid from reaching the edges of the product; provide conforming means that permit the end regions of the napkin to conform and adapt to the surface of the body in use. The channel impressions must also be located in the central region, as well as in the end regions, to sustain the path of wicking. Such centrally located channels, in use, become hinges that prevent the napkin's central region from remaining falt beneath the relatively flat longitudinal region of the labia majora, thereby causing the napkin to bend concavely away from the labia majora.

Curved compression lines as disclosed in EP 0 136 524 and WO 91/14415 are not generally perpendicular but generally parallel to a central longitudinal axis of an absorbent product. The articles of the WO 91/14415 are as such relatively rigid in order to withstand a deformation force. They comprise a compressed bottom absorbent layer of predetermined rigidity or stiffness which serves as means for preventing undesirable deformation of the article.

The EP 0 621 081 A1 solely teaches to form fluid barriers due to which recesses in the garment side surface develop. The fluid barriers are not adapted to function as a stiffened element. The fluid barriers have a concentric contiguous form. Therefore the absorbent product does not provide a region wherein the absorbent product is preferentially bend.

It is in object of the present invention to provide a disposable absorbent product for use in feminine hygiene which is adapted to cover and closely fit the perineal area of the wearer.

It is another object of the present invention to provide an absorbent article having a central region which is adapted to cover the vestibule and labia majora of the wearer, a forward end region adapted to cover the mons pubis of the wearer, and a rearward end region adapted to cover the posterior perineum of the wearer.

To solve the object in accordance with the present invention, there have been provided protective absorbent products for use by a woman for feminine hygiene, and more particularly improved sanitary protection products such as sanitary napkins, panty liners and incontinence products, that have conforming means, comprising stiffened elements and unstiffened regions in their end regions that, in use, facilitate the product's ability to fit close and conform to the body, resist transverse bunching and prevent leakage of fluid from the edges. Thus, the absorbent products of this invention, have a central region which is adapted to cover and fit the labia majora and the vestibule contained within, and end regions adapted to cover and fit the body's anterior region of the mons pubis as well as the body's posterior region consisting of the posterior perineum, respectively.

The absorbent product of this invention comprises as defined by the features of claim 1 a liquid permeable body facing layer, a liquid impermeable garment facing layer, an absorbent structure therebetween defining an absorbent element. The absorbent element has longitudinal edges opposite each other defining a width dimension, a central longitudinal axis parallel to the longitudinal edges, transverse edges opposite each other defining a length dimension, and a thickness dimension. The absorbent element further comprises a central region, a first or anterior end region and a second or posterior end region. The central region has longitudinal edges coincident with the longitudinal edges of the absorbent element as well as first and second distal ends opposite each other defining an area that is sufficient to cover at least the woman's vestibule and labia majora in use. The first or anterior end region, extending from the first distal end of the central region, is adapted to cover at least a portion of the woman's mons pubis in use. The second or posterior end region, extending from the second distal end of the central region, is adapted to cover at least a portion of the woman's posterior perineum in use.

The absorbent product of this invention further comprises at least one conforming means, contained within the at least one end region, the conforming means comprising a nonstiffened region adjacent the distal end of the central region and adjacent to a stiffened element, the stiffened element being located inward of the transverse and longitudinal edges of the absorbent element. The non-stiffened region extends transversely across the end region, generally perpendicular to the central longitudinal axis of the absorbent product from one longitudinal edge of the absorbent element to the opposite longitudinal edge of the absorbent element. The stiffened element extends transversely across the end region, perpendicular to the central longitudinal axis of the absorbent product, at least at its intersection with that axis, and centrally occupies at least 50% of the width of the absorbent element. The stiffened element further includes at least one perpendicular component extending longitudinally across at least a portion of the end region, the perpendicular component extending longitudinally beyond at least a portion of the lateral component. The stiffened element, in use, resists transverse bunching and in combination with the nonstiffened region, in use, enables the end region of the absorbent product to preferentially bend and to closely fit the body. The stiffened element may also act as a barrier to fluid wicking and guide the fluid so that it is retained within the confines of the absorbent element. The conforming means thereby provides an axis of bending, that coincides with a transverse axis of the napkin, a resistance to bending and compression orthogonal to that axis, i.e., along the longitudinal axis of the napkin and a relatively unconstrained region that may puff and cuff outward to fit and conform to the body. The stiffened element may optionally be adapted to prevent leakage of fluid from the edges, as hereinafter described. The stiffened element may of itself be a bending means, or may include a separate bending means, such as an embossed channel, or may comprise a projection, pleat, slit, hinge means or thinned area, that provides an axis of flexibility, coincident with the axis of bending of such bending means as well as providing stiffening and compression resistance orthogonal to the axis of bending.

The center region may optionally contain one or more stiffening means, such as extra absorbent material, a sphagnum-moss containing insert, an embossed channel having a component parallel to the central longitudinal axis, and combinations thereof. Embossed channels are preferably located between the central longitudinal axis and the longitudinal edge, provided of course that the stiffening means in the central region is spaced apart from the stiffened element in the end region. The stiffening means maintains the center of the absorbent element in a relatively flat profile along the longitudinal axis, and resists bending of the absorbent element transversely to that axis, so as to effectively conform to the body in that region, resist transverse bending and bunching and thereby prevent leakage of fluid from the absorbent element.

In accordance with another embodiment of the present invention, the absorbent product may also have cuffs, undergarment wrapping wings or tabs that are either attached to the edges of the absorbent element or attached inwardly of the longitudinal edges of the absorbent element. Such cuffs, wings or tabs help maintain the surface of the central region of the napkin, flat and spread open, along the napkin's longitudinal axis. Additionally, such cuffs or wings or tabs have been found to cooperate with the conforming means of this invention to provide full and reserve coverage of and enhanced conformability to the body, by the absorbent product, in use. In a preferred embodiment the wings or tabs are attached inwardly of the longitudinal edges of the absorbent element, such wings or tabs gather the edges of the undergarment under the absorbent element without diminishing the strike zone that is available to capture body fluids.

Preferred embodiments are illustrated with the drawing in which:
Fig. **1** is a perspective view of an hourglass shaped sanitary napkin, however not of this invention, comprising conforming means each having one stiffened element and an adjacent unstiffened region in each of the end regions.
Fig. **2** is a cross sectional view taken through axis A-A of Fig. **1**, to illustrate the stiffened element of the invention, which is in this embodiment an embossed channel.
Fig. **3** through **7** are cross sectional views taken through axis A-A of Fig. **1** to illustrate alternative examples of configurations of the embossed channel stiffened element of the invention.
Fig. **8** is a plan view of a rectangular shaped sanitary napkin, however not of this invention, comprising one end region with a conforming means having two stiffened elements; and a second end region with a conforming means having one stiffened element
Figs. **9** through **10** are cross sectional views taken through axis A-A of Fig. **8**, through the end region having two stiffened elements, of examples of alternative configurations of the two stiffened elements of the invention.
Fig. **11** is a plan view of a rectangular shaped sanitary napkin of this invention comprising a stiffened element that may be part of the conforming means in each of the end regions.
Fig. **12** is a cross sectional view through axis A-A of a stiffened element shown in Fig. **1**, wherein the stiffened element comprises a reinforcing strip of additional material.

The discussion is primarily directed to describing the present invention and its use as a unique absorptive structure for feminine hygiene, with examples being given as sanitary napkins. However, this invention is meant to apply equally well, with such necessary modifications as will be known to those skilled in the art, to products such as panty liners, incontinence products and diapers.

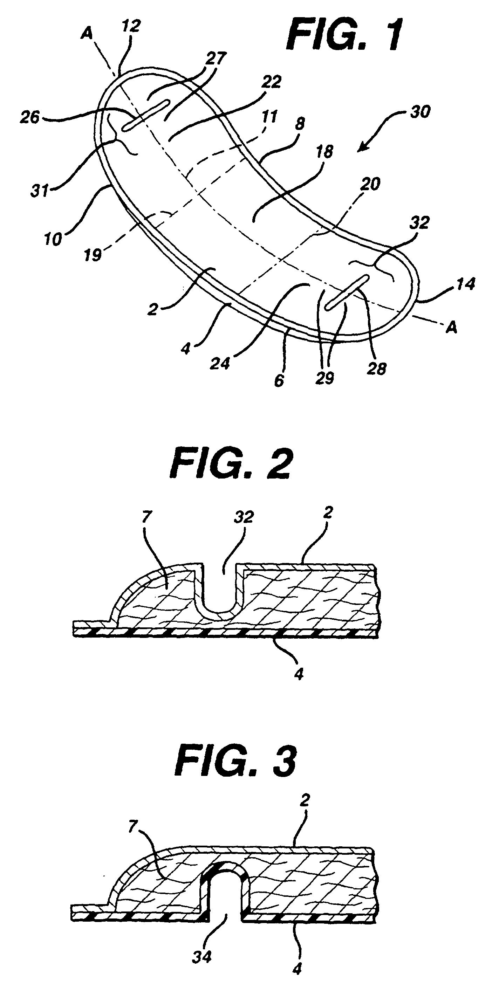

Referring now to the drawings in detail. Wherein like numerals indicate the same elements throughout the view, Fig. **1** is a perspective view of a sanitary napkin **30** having comprising a liquid permeable body facing layer **2**, a liquid impermeable garment facing layer **4**, an absorbent structure (not shown) therebetween defining an absorbent element 6 has:
Longitudinal edges 8,10 opposite each other defining a width dimension, a central longitudinal axis **11** parallel to longitudinal edges **8**, **10**, transverse edges **12**, **14** opposite each other defining a length dimension, and a thickness dimension;
A central region **18** having a length which is sufficient to cover the woman's vestibule and labia major in use, the central region **18** having longitudinal edges **8, 10** coincident with longitudinal edges **8, 10** of absorbent element **6**; and first and second distal ends **19, 20** opposite each other;
a first end region **22**, adjacent to and extending from first distal end **19** of central region **18**, the first end region **22** being adapted to cover at least a portion of the woman's mons pubis in use;
a second end region **24**, adjacent to and extending from the second distal end **20** of central region **18**, the second end region **24** being adapted to cover at least a portion of the woman's posterior perineum in use; and
conforming means, 31, 32, contained within at least one of the respective end regions **22, 24**, the conforming means **31, 32** comprises a nonstiffened region **27, 29** respectively, adjacent to distal ends **19, 20** of central region **18** and adjacent to a stiffened element **26, 28,** the stiffened elements **26, 28** being respectively located inward of transverse edges **12, 14** and longitudinal edges **8, 10** of end regions **22, 23**.

Nonstiffened regions **27**, or **29**, extend transversely across end regions **22**, or **24** respectively, generally perpendicular to central longitudinal axis **11** of absorbent element **6**, and are immediately adjacent respective stiffened elements **26,** or **28,** respectively to conform to the curved surfaces of the body. Such nonstiffened regions are located immediately adjacent all sides, edges and ends of the stiffened elements and are also located between stiffened elements when more than one stiffened element is present in the end region.

Stiffened elements **26, 28** extend transversely across end regions **22, 24**, inward of the longitudinal edges **8** and **10**, and are generally perpendicular to central longitudinal axis **11** of absorbent element **6**, at least at its intersection with longitudinal axis **11** and centrally occupying at least 50% of the width of absorbent element **6**. Stiffened elements **26**, **28** are any means that rigidify the structure against transverse compression and bunching in use and that slow, channel or halt the migration of fluid that is wicking from any nonstiffened regions **27**, **29**, containing fluid, adjacent one side of such elements, to other nonstiffened regions on sides opposite the stiffened elements, not yet containing fluid. Stiffened elements **26**, **28** may for example be embossed channels, densified regions, projections above the body facing layer, additional material strips reinforcing at least body facing layer **2**, resin-containing regions of the absorbent structure, hotmelt-containing regions of the absorbent structure, superabscrbent containing regions of the absorbent structure, or combinations thereof. Stiffened elements **26, 28** also cooperate to confine fluid within absorbent element **6,** such elements being located inwardly of transverse edges **12**, **14** and longitudinal edges **8**, **10** of end regions **22**, **23**. Stiffened elements **26**, **28** also resist transverse compression and bunching, in use, being located transversely across the end region, generally perpendicular to the central longitudinal axis of the absorbent product, at least at its intersection with that axis, and centrally occupying at least 50% of the transverse width of the end region. It is preferred that stiffened elements have at least one lateral component located transversely across an end region generally perpendicular to a central longitudinal axis of the napkin, at least at its intersection with that axis, and centrally occupy at least 50 % of the transverse width of the end region and less than 95 % of the transverse width of the end region.

Stiffened element **26**, **28** in use, resist transverse compression and bunching; and may bend along axes of bending, if such are present as in embossed channels, the axes of bending coinciding with transverse axes of napkin **30**. Stiffened elements **26, 28** may also act as barriers to fluid wicking and guide fluid so that it is retained within the confines of absorbent element **6**.

Nonstiffened regions **27**, **29**, in use, enable the absorbent product to preferentially bend transversely to the longitudinal axis of the absorbent element and to closely fit the body along the length of napkin **30**. Conforming means **31**, **32** thereby provide both: stiffened elements **26**, **28**, that may bend along their axis of bending, that coincides with a transverse axis of napkin **30**, and resists bending and compression orthogonal to longitudinal axis **11** of napkin **30**; and a nonstiffend region that preferentially bends to permit the end region to conform to the body. The stiffened element additionally may prevent leakage of fluid from the edges.

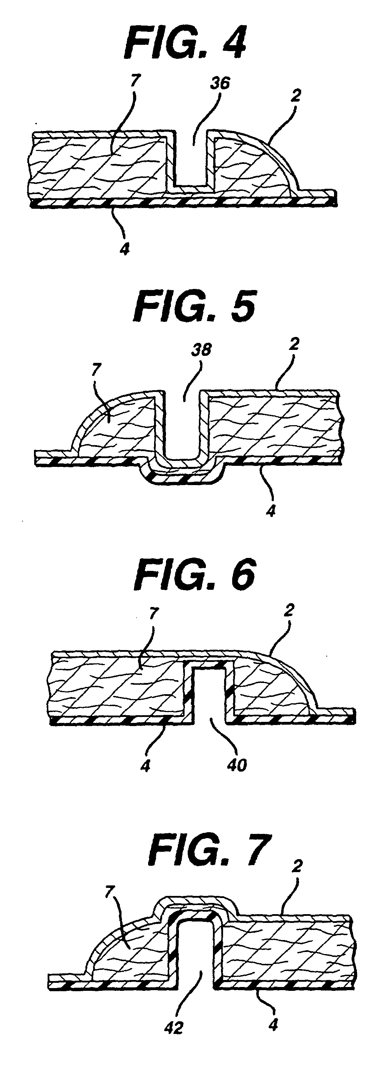

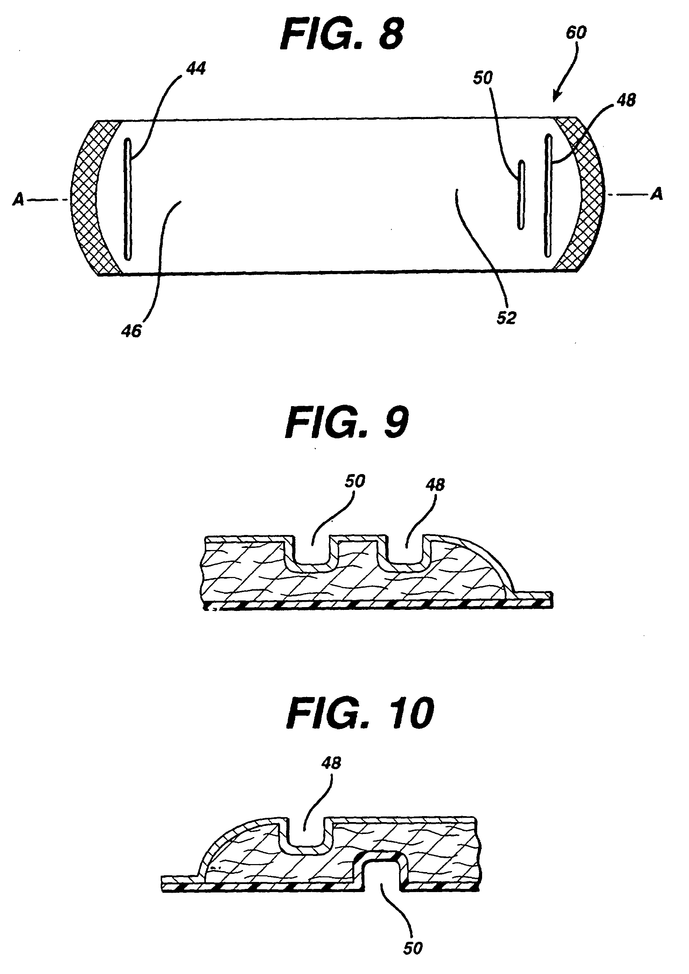

Embossed channels are a preferred embodiment of the stiffened elements of this invention. When stiffened element **26**, **28** are themselves relatively bendable along their length, as for example in embossed channels, they comprise a hinge means, that enables napkin **30** to also bend along stiffened elements **26**, **28** and to conform to curved surfaces of the body. Embossed channels as stifened elements **26**, **28** also cooperate to confine fluid within absorbent element **6**, such elements being located inwardly of transverse edges **12**, **14** and longitudinal edges **8**, **10** of end regions **22**, **23**. Embossed channels stiffen the napkin in directions perpendicular to the channels along their entire length. Therefore, channels that are aligned transversely in napkin **30** will resist bunching in a transverse direction; and channels that have a longitudinally oriented component will resist compression in the longitudinal direction of napkin **30**. Thus, stiffened elements **26**, **28** as embossed channels, and their adjacent nonstiffened regions **27**, **29**, cooperate to bend, conform and closely fit the body, to resist transverse compression and bunching, and at times longitudinal compression; and to prevent leakage of fluid from napkin's **30** edges. Embossed channels may be impressed into the absorbent structure alone of napkin **30**, either from the body facing side or from the garment facing side of the absorbent structure. Embossed channels may be impressed into napkin **30** either from the body facing layer **2** or from the garment facing layer **4** through the any part of the thickness of absorbent element **6** and even up to protruding beyond the opposite facing layer; while taking in all such executions, not to perforate the garment facing layer, thereby destroying its fluid impermeability. Examples of such possibilities are illustrated in Figs. **2** through **7**, as follows: Fig. **2** is a cross sectional view taken through axis A-A of Fig. **1**, and limited to end region **24**, wherein either of stiffened elements **26** or **28** is an embossed channel **32** that is impressed downward into body facing layer **2** and absorbent structure **7** beneath. Fig. **3** is a cross sectional view taken through axis A-A of Fig. **1**, and limited to end region **24**, wherein either of stiffened elements **26** or **28** is an embossed channel **34** that is impressed upward into the garment facing layer **4** and absorbent structure **7** above. Fig. **4** is a cross sectional view taken through axis A-A of Fig. **1**, and limited to end region **24**, wherein either of stiffened elements **26** or **28** is an embossed channel **36** that is impressed downward into body facing layer **2**, absorbent structure **6** and garment facing layer **4** beneath. Fig. **5** is a cross sectional view taken through axis A-A of Fig. **1**, and limited to end region **24**, wherein either of stiffened elements **26** or **28** is an embossed channel **38** that is impressed downward into body facing layer **2**, absorbent structure **7** and garment facing layer **4** and protrudes beyond garment facing layer **4**. Fig. **6** is a cross sectional view taken through axis A-A of Fig. **1**, and limited to end region **24**, wherein either of stiffened elements **26** or **28** is an embossed channel **40** that is impressed upward into garment facing layer **4**, absorbent structure **7** and body facing layer **2** above. Fig. **7** is a cross sectional view taken through axis A-A of Fig. **1**, and limited to end region **24**, wherein either of stiffened elements **26** or **28** is an embossed channel **42** that is impressed upward into garment facing layer **4**, absorbent structure **7** and body facing layer **2** and protrudes beyond body facing layer **2**. Napkins may have more than one stiffened element in an end region; and the number and type of stiffened elements in each end region do not need to be the same. Thus, Fig. **8** shows a napkin **60** however not of this invention having one stiffened element **44** in end region **46** two stiffened elements **48**, **50** in end region **52**. Figs. **9** and **10** are cross sections of two alternative configurations, taken through axis A-A of napkin **60**, shown in Fig. **8**, and limited to end region **52.**

Stiffened elements may have any shape or configuration and may comprise continuous segments, discontinuous segments, straight segments, curved segments or combination thereof. It is preferred that the elements be continuous. If the stiffened elements are discontinuous, it is preferred that the segments not be spaced apart from each other by more than 6.35 mm (0.25 inches) in order to maintain good fluid barrier properties and deformation and bunching resistance for the absorbent element. It is preferred that stiffened elements have at least one perpendicular component that is generally orthogonal to the lateral component and generally parallel to the central longitudinal axis of the absorbent product, and with the perpendicular component being closer to the longitudinal edge than it is to the central longitudinal axis. The perpendicular component need not be strictly orthogonal to the lateral component. It may also be at some acute or obtuse angle to the lateral component, and may extend towards the transverse edge, towards the central region, or both. It is important that the stiffened element be located inwardly of the transverse edges of the end region of the absorbent element, and is preferably approximately between 3.175 mm (0.125 inches) and 63.5 mm (2.5 inches) inwardly of the transverse edges of the end region.

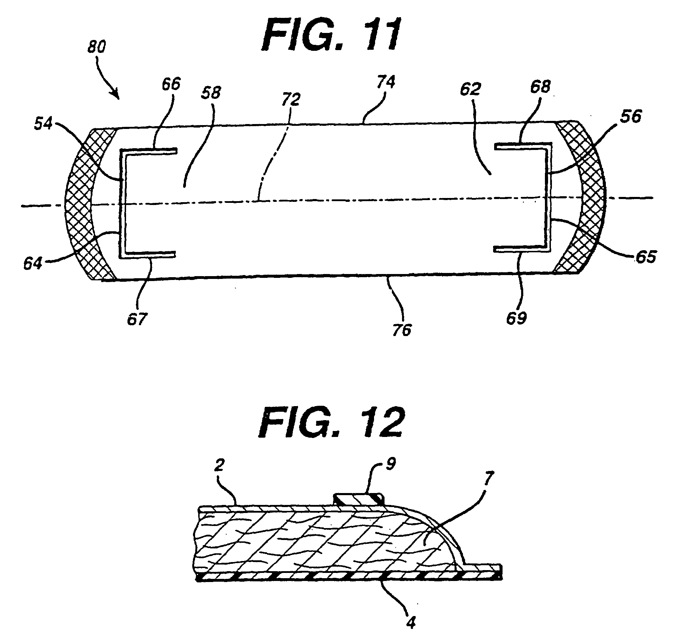

Fig. **11** shows a napkin **80** of this invention having stiffened elements **54**, **56** respectively, in end regions **58, 62,** wherein each stiffened element comprises a lateral component **64**, **65** located transversely across respective end regions **58, 62** and perpendicular components **66**, **67**, **68**, **69**, that are orthogonal to lateral components **64**, **65** and parallel to the central longitudinal axis **72** of the napkin **80**; and are closer to the longitudinal edges **74**, **76** than they are to central longitudinal axis **72**.

Fig. **12** shows a cross section through axis A-A of Fig. **1**, wherein the stiffened region is a reinforcing strip **9** comprised of additional material. The additional material may be, for example, comprised of the same materials as that of the body facing layer, the absorbent structure, components of the absorbent structure, the garment facing layer, other plastic or fibrous materials, and combinations thereof. The center region of the absorbent product of this invention may optionally contain a stiffening means, spaced apart from the at least one stiffened element. Examples of stiffening means are such as extra or thicker absorbent material, a sphagnum moss-containing insert, a rigid insert, a semirigid insert, embossed channels, or combinations thereof. The stiffening means is located in the center region of the sanitary napkin and is spaced apart from the stiffened element so as not to compromise the conforming and fit to the body provided by the unstiffened region of the conforming means. The stiffening means maintains the center region of the absorbent element in a relatively flat profile along the longitudinal axis, in order to best cover and closely fit the body in that region and to resist transverse bending and bunching, and thereby prevent leakage of fluid from the absorbent element. The effect and benefit of such stiffening means is described in commonly assigned US Patent Application Serial No. 08/198, to McCoy, "Body Conforming Absorbent Article",

Examples of semirigid inserts include sphagnum moss and plastic sheets. Rigid inserts may be obtained by using thicker forms or laminates of the materials used for semirigid inserts. Embossed channels, in the form of at least one deeply embossed channel, either alone or in combination with other stiffening means, are a particularly preferred form of stiffening means; and are preferably oriented along the central longitudinal axis, thereby providing at least one longitudinal channel, and located anywhere between and including the central longitudinal axis and the longitudinal edge of the absorbent element of the absorbent product of this invention. Such channels may be straight or curved, curving outwardly from or inwardly toward the longitudinal axis. Other more complex curves, as may be conceived for such channels, may be used. It is further preferred that the distance between the stiffened element and each longitudinal channel of the stiffening means be greater than the distance between the stiffened element and any of the transverse and longitudinal edges adjacent the stiffened element, so as not to compromise the conforming and fit to the body provided by the unstiffened region of the conforming means; and to maximize the protection from leakage to the longitudinal edges afforded by both the longitudinal channels and the stiffening elements.

Napkins of this invention may be provided with a means for attaching it to the undergarment such as adhesive, protected by release paper until use, or by mechanical attachments such as a hook and loop assembly, clasp assembly, hinge assembly or by combinations thereof. Release paper may be eliminated if the napkins of this invention are packaged in a wrapper that has a napkin facing surface that is of itself releasable from adhesive by virtue of being coated or formulated with a release substance such as silicone or fluorocarbon or by being physically altered, such as by embossing, to reduce its contact with the adhesive.

Body facing layers may be comprised of at least a single layer or combinations of perforate film or foam or of a fabric such as is represented by wovens, knits and nonwovens, the nonwovens being represented by those such as are represented by the processes that produce spunbond, meltblown, needlepunched, thermobonded, chemical binder bonded, powder bonded, solvent bonded and hydroentangled fabrics. Perforate films may be such as those whose surfaces are flat or embossed, the bosses being of: micro size that may be visually detected as a matte finish or tactilely detected as having a silky, smooth feel; or of micro size where the individual bosses may be unaidedly seen or felt. The perforations may be two-dimensional, being essentially restricted to the plane of the film, or three-dimensional, where the film structure that defines and supports the perforations extends from the plane of the film and beyond, i.e., above or below, the plane of the film. Examples of combinations of the materials are those that may be forced by physical unadhered lamination, adhesive or thermal lamination or by interpenetrating lamination, e.g., fibrous webs laminated to perforate films. The foams may be reticulated or non reticulated. The surfaces of body facing layers, i.e., outer facing surface, which is the facing surface of the absorbent structure, may be hydrophobic, hydrophilic, or one surface may be hydrophobic and the other hydrophilic or having gradients of hydrophobicity to hydrophilicity from one surface to the other.

The body facing layer of the absorbent product of this invention may cover any part or all of the upper surface, that is the body facing surface, of the absorbent structure. It may alternatively wrap the absorbent structure partly or entirely around. Part wrapping of the absorbent structure is exemplified by the permeable body facing layer covering the top of and the sides of the absorbent structure. The body facing layer may be fixed or otherwise adhered to the surface of the absorbent structure overall or in discrete zones of attachment. Depending on the degree of coverage and wrapping of the absorbent structure by the body facing layer, it may be adhered to itself for example in an overlapping configuration at the bottom of the absorbent structure. The garment facing layer may cover at least the entire bottom surface, that is the garment facing surface, of the absorbent structure. It may also wrap around to cover the sides of the absorbent structure and even part of the body facing surface of the absorbent structure.

The absorbent structure may be comprised of absorbent materials that accept, transfer, distribute, store and retain fluid as well as prevent fluid from exiting the absorbent product. The absorbent structure may be a simple absorbent such as woodpulp, which may contain stabilizing components such as synthetic fibers, that are used as such, to form a bridging matrix; or by being thermobondable, are fused to themselves and to the woodpulp to form a dimension stabilizing structure. The synthetic fibers may be either hydrophilic, such as rayon, or hydrophobic such as polypropylene and polyester. The synthetic fibers may be made more wettable by treatment with a wetting agent such as a surfactant, by caustic etching of fibers such as polyester, by incorporating wettable polymers such as polyethylene oxide or polyvinyl alcohol within the fiber polymer formulation, by grafting the fiber surface with wettable reactants and by exposing the fiber to corona discharge. The peripheral profile of synthetic fibers may be of any shape, e.g., round, oval, multi-lobal. The synthetic fibers may also contain grooves, channels or bores; and may be pitted or perforated. The absorbent core may also contain auxiliary absorbents such as rayon or cotton fibers, sphagnum moss and superabsorbent fibers or particles.

Absorbents such as sphagnum moss, in board or in compressed layer form, may function additionally as compression resisting or deformation resisting structures or to help maintain a flat or raised product profile. Absorbents in board form may be made flexible and conforming by tenderizing by means of passing the board through a corrugating or embossing process. The woodpulp itself may also be comprised, at least in part, of any of wet crosslinked, dry crosslinked, chemically stiffened or curly fibers. The synthetic fibers and auxiliary absorbents may be present homogeneously throughout the absorbent core, in discrete layers or in continuous or discontinous concentration gradients. The absorbent core may also contain foam in the form of layers or particles, the foam being either hydrophobic or hydrophilic, depending on its location and function in the product, e.g., absorbing, cushioning, deformation resisting and compression resisting. The absorbent core may be uncompressed, compressed, or otherwise densified, at least in part. Compression and densification may be homogeneous throughout the absorbent core or in discrete layers or in continuous or discontinuous gradients of density.

The absorbent structure may contain, in addition to the absorbent core, a transfer layer, which is a low density fluid accepting and fluid releasing layer, usually located between the absorbent core and the permeable cover. The transfer layer may be comprised of relatively less hydrophilic materials and structures, than is contained in the absorbent core, such as of webs of meltblown polypropylene or polyester fibers. Such webs may also contain woodpulp entrained within. Transfer layers may also be comprised of low density, highloft nonwoven webs comprised of woodpulp and synthetic fibers such as polyethylene, polypropylene, polyester, polyacrylonitrile and polyamide. Such highloft webs may be bonded with chemical binders or by thermal means such as by through-air bonding.

The garment facing layer may be fixed or otherwise adhered to the surface of the absorbent structure overall or in discrete zones of attachment. The garment facing layer may be adhered to the body facing layer in an overlapping configuration for example parallel to the sides of the absorbent element or parallel to the bottom of the napkin or in a flange seal extending from the sides of the absorbent element. When the body facing layer and garment facing layer are adhered to each other in a flange seal, the body facing layer may additionally be wrapped around the flange seal about the body facing layer; or the garment facing layer may additionally be wrapped around the flange seal about the body facing layer.

The impermeable garment facing layer may be of any flexible material that prevents the transfer through it of fluid but does not necessarily prevent the passages of gases. Commonly used materials are polyethylene or polypropylene films. Other materials that may be used as impermeable barriers may be chosen from films of polyesters, polyamides, ethylene vinyl acetate, polyvinyl chloride, polyvinylidene chloride, cellophane, nitrocellulose and cellulose acetate. Co-extruded and laminated combinations of the foregoing, wherein such combinations are permitted by the chemical and physical properties of the film, may be used. Fluid impermeable nonreticulated foams and repellent treated papers may also be used. Films that are fluid barriers, but permit gases to transpire, i.e., "breathable films", may be used. These may be chosen from polyurethane films and from micro-porous films, where micro-porosity is created by ionizing radiation or by leaching out of soluble inclusions using aqueous or nonaqueous solvents. Fabrics whose surfaces have been made repellent or whose pores are small by virtue of close packing of fibers, or whose pores have been reduced in size by closing off large liquid admitting pores, may also be used alone, or together with breathable films, as breathable barriers.

The absorbent element may be of many different shapes and sizes, depending on the requirements of the user with reference to her anatomy, menstrual flow volume and intensity, duration of wear and the part of the day or night the product is being worn. For example, absorbent elements may be of generally rectangular shape, with generally straight or somewhat curved longitudinal and transverse edges, the corners defining the intersection of such edges being either square or rounded. Absorbent elements may also be narrower in the central region than in the end regions, being of for example, of dogbone or hourglass shape; or they may be wider in the central region than in the end regions, being for example of oval or round shape. The end regions may or may not be symmetrical about the central region. The end regions may or may not be the same shape or size as each other. The absorbent elements of this invention will have the following approximate dimensional ranges: a length dimension range of between 101.6 and 330.2 mm (4 and 13 inches), a width dimension of between 38.1 and 101.6 mm (1.5 and 4 inches), and a thickness dimension of between 1.27 and 25.4 mm (0.05 and one inch). The thickness of the absorbent elements of this invention may be uniform throughout the expanse of the absorbent element or, for throughout the expanse of the absorbent element or, for the purpose of specific fit, flexibility and absorbency requirements, the absorbent element may be thicker in some regions than in others. For example, a particularly preferred thickness profile is an absorbent Structure that is thicker in the central region than it is in the end regions.

The absorbent products of this invention may also comprise auxiliary components that may add to the functional, comfort and aesthetic properties of the products such as gasketing cuffs or garment attaching tabs, the tabs being also known as wings or flaps. Such cuffs, wings or tabs help maintain the surface of the central region of the napkin, flat and spread open, along the napkin's longitudinal axis. Additionally, such cuffs or tabs or wings synergistically cooperate with the conforming means of this invention to provide full and reserve coverage of and conformability to the body, by the absorbent product, in use. This is true of cuffs and especially true of tabs or wings that are attached inwardly of the longitudinal edges of the absorbent element, such tabs or wings providing gathering of the crotch of the undergarment without diminishing the strike zone that is available to capture body fluids.

The cuffs and tabs or wings, as given below in the examples of sanitary napkins as absorbent products, may be attached to or be extensions of the sanitary napkin of the napkin's body facing side, napkin sides or garment facing side. The cuffs and tabs or wings may be comprised of materials that are different from those of the napkin, or may be comprised of materials of which the napkin is made, or of combinations of the different materials and the materials of which the napkin is made. When the cuffs and tabs or wings are made of the materials of which the napkin is made, the materials may be attached to the napkin or be formed of extensions of the napkin's materials. Examples of constructions of cuffs and tabs or wings are: where the permeable cover material and the impermeable barrier material are attached to themselves, or to each other, along the periphery of the cuff or tab or wing structure, in discrete areas, or over their entire area of contact. The cuffs and tabs or wings may have laminated, between the cover and barrier materials, extensions of part or all of the absorbent body, for example extensions of one or both of the transfer layer and a portion of the absorbent core. The tabs or wings may be provided with adhesive, protected with release paper, for attachment to the undergarment, or they may be attached by mechanical attachments such as a hook and loop assembly, clasp assembly, hinge assembly or by combinations thereof. The cuffs and tabs or wings may also contain additional materials to make them thick and cushioning and may also contain, separately or additionally, flexible, stretchable or elastic materials. Such materials have the effect, on the cuffs and tabs or wings, and at times on the napkin itself, of gathering, curving or causing to them to conform to the body and the garment. Embodiments of cuffs and tabs or wings such as are described in the following commonly assigned US Patents and Patent Applications: US Patent No. 4,940,462 to Salerno, "Sanitary Napkin with Expandable Flaps"; US Patent No. 5,490,847 to Correa et al; "Disposable Sanitary Napkin", US Patent No. 4,900,320 to McCoy, "Sanitary Napkin with Panty Gathering Flaps"; US Patent Application, Serial No. 08/198,809 to McCoy; "Body Conforming Absorbent Article"; US Patent Article Having Preformed Compliant Cuffs; US Patent Application, Serial No. 08/552, 876 to Salerno et al, "Absorbent Article Having Compliant Cuffs"; and US Patent Application, Serial No. 08/552,881 to Salerno et al, "Stabilized Absorbent Article".

## Claims

1. An absorbent product (30) adapted to be worn in an undergarment by a woman for feminine hygiene comprising a liquid permeable body facing layer (2), a liquid impermeable garment facing layer (4), an absorbent element therebetween the absorbent product (6) having:
longitudinal edges (8, 10) opposite each other defining a width dimension, a central longitudinal axis (11) parallel to the longitudinal edges (8, 10), transverse edges (12, 14) opposite each other defining a length dimension;
a central region (18) having first and second distal ends (19, 20) opposite each other defining a length that is sufficient to cover a woman's labia majora in use;
a first end region (22), extending from the first distal end (19) of the central region (18) and being adapted to cover at least a portion of a woman's mons pubis in use;
a second end region (24), extending from the second distal end (29) of the central region (18) and being adapted to cover at least a portion of a woman's posterior perineum in use; and **characterised in that** the absorbent product further comprises
at least one conforming means (31, 32), contained within at least one end region (22, 24), comprising a non stiffened region (27, 29) adjacent the distal end (19, 20) of the central region (18) and adjacent to a stiffened element (26, 28), the stiffened element (26, 28) being located inward of the transverse and longitudinal edges (12, 14; 8, 10) of the absorbent product (6), the stiffened element (26, 28) having a lateral component (64, 65) extending transversely across the at least one end region (22, 24) perpendicular to the central longitudinal axis (72) of the absorbent product (6) and centrally occupying at least 50% of the width of the absorbent product (6);
the stiffened element (26, 28) further including at least one perpendicular component (66, 67, 68, 69) extending longitudinally across at least a portion of the end region, the perpendicular component extending longitudinally beyond at least a portion of the lateral component;
wherein the absorbent product preferentially bends in the non-stiffened region (27, 29) and the stiffened element (26, 28) resists transverse bunching.

2. The absorbent product of claim 1, wherein the conforming means (3I, 32) contains at least one axis, perpendicular to the central longitudinal axis, and the bending resistance of the absorbent element (6) along the at least one axis is not greater than compared to the absence of the stiffened element (26, 28) in the absorbent element (6) of the conforming means (31, 32).

3. The absorbent product of claim 1 or 2, wherein the absorbent product is a sanitary napkin, panty liner, or an incontinence product.

4. The absorbent product of any of the preceding claims, wherein the stiffened element (26, 28) also comprises at least one of an embossed channel (32), a densified region, a projection above the body facing layer (2), an additional material strip reinforcing at least the body facing layer (2), a resin containing region of the absorbent structure (7), a hotmelt containing region of the absorbent structure, or combinations thereof.

5. The absorbent product of any of the preceding claims, wherein the stiffened element centrally occupies less than 95% of the transverse width of the end region.

6. The absorbent product of claim 5, wherein a centered outer rim of 5% of the transverse width of the end regions is free from occupation of the stiffened element.

7. The absorbent product of claim 4, wherein the stiffened element comprises at least an embossed channel (38) impressed into the absorbent structure, in particular wherein the embossed channel (38) is impressed downward into the body facing permeable layer (2) and the absorbent structure (7) beneath.

8. The absorbent product of any of the preceding claims, wherein the embossed channel is impressed upward into the garment facing layer and the absorbent structure above.

9. The absorbent product of any of the preceding claims, wherein the embossed channel (36) is impressed downward into the body facing layer (2), the absorbent structure (6) and the garment facing layer (4) beneath.

10. The absorbent product of any of the preceding claims, wherein the embossed channel (38) protrudes beyond the garment facing layer (4).

11. The absorbent product of any of the preceding claims, wherein the embossed channel (34) is impressed upward into the garment facing layer (4), the absorbent structure (7) and the body facing layer (2) above.

12. The absorbent product of claim 11, wherein the embossed channel (42) protrudes beyond the body facing layer (2).

13. The absorbent product of any of the preceding claims, having at least one embossed channel (48) impressed downward into the body facing layer (2) and the absorbent structure (6) beneath and at least one embossed channel (50) impressed upward into the garment facing layer (45) and the absorbent structure (6) above.

14. The absorbent product of any of the preceding claims, wherein the length dimension is in the approximate range of between 101.6 and 330.2 mm (4 and 13 inches), the width dimension is in the approximate range of between 38.1 and 101.6 mm (1.5 and 4 inchs).

15. The absorbent product of any of the preceding claims, wherein the stiffened element is located approximately between 3.175 mm (0.125 inches) and 63.5 mm (2.5 inches) inwardly of the edges of the end region.

16. The absorbent product of any of the preceding claims, wherein the stiffened element (54, 56) comprises a lateral component (64, 65) located transversely across the end region (58, 62) and additionally at least one perpendicular component (66, 67, 68, 69) that is orthogonal to the lateral component (64, 65) and parallel to the central longitudinal axis (72) of the absorbent product (80), and is closer to the longitudinal edge (74, 76) than it is to the central longitudinal axis (72).

17. The absorbent product of any of the preceding claims, wherein the stiffened element is of continuous segments, discontinuous segments, straight segments, curved segments or combination thereof.

18. The absorbent product of any of the preceding claims, which is of rectangular shape in the plan view, and wherein the longitudinal edges are parallel to and equidistant from each other.

19. The absorbent product of any of the preceding claims, wherein at least part of the center region has a width that is smaller than the transverse width of at least one of the end regions.

20. The absorbent product of any of the preceding claims, which additionally has at least one garment attaching tab which is attached to one of the longitudinal edges of the absorbent element.

21. The absorbent product of any of the preceding claims, which additionally has at least one garment attaching tab which is attached inwardly of one of the longitudinal edges of the absorbent element.

22. The absorbent product of any of the preceding claims, which additionally has at least one cuff (136, 137) which is attached to one of the longitudinal edges (140, 141) of the absorbent element (160).

23. The absorbent product of any of the preceding claims, which additionally has at least one cuff (136, 137) which is attached to one of the longitudinal edges (140, 141) of the absorbent element (160) and at least one garment attaching tab (161, 162) which is attached inwardly of one of the longitudinal edges (165, 166) of the absorbent element (135).

24. The absorbent product of any of the preceding claims, wherein the absorbent structure is thicker in the central region (1529 than it is in the end regions (146, 147).

25. The absorbent product of any of the preceding claims, wherein the absorbent structure is comprised of an absorbent core.

26. The absorbent product of any of the preceding claims, wherein the absorbent structure is comprised of a transfer layer and an absorbent core.

27. The absorbent product of claim 25 or claim 26, wherein the material comprising the absorbent core is selected from the group consisting of woodpulp, tissue, synthetic fibers treated to make them hydrophilic, hydrophobic synthetic fibers in mixtures of hydrophilic fibers, sphagnum moss, superabsorbent particles, superabsorbent fibers and combination thereof.

28. The absorbent product of claim 1, wherein the product comprises at least one stiffening means in the center region spaced apart from the stiffened element and wherein the stiffening means facilitates the ability of the absorbent product to resist longitudinal bending.

29. The absorbent product of claim 28, wherein the stiffening means comprises at least one of an embossed channel, an extra thickness of absorbent material, a rigid insert, a semirigid insert or combinations thereof.

30. The absorbent product of claim 28 or claim 29, wherein the embossed channel is a longitudinal channel located between the central longitudinal axis and the longitudinal edge.

31. The absorbent product of claim 30, wherein the longitudinal channel is straight.

32. The absorbent product of claim 30, wherein the longitudinal channel is curved, curving inwardly toward the longitudinal axis.

33. The absorbent product of claim 32, wherein the longitudinal channel is curved, curving inwardly toward the longitudinal axis.

34. The absorbent product of claim 29, wherein the semirigid insert contain sphagnum moss.

35. The absorbent product of claim 29, wherein the semirigid insert is a plastic sheet.

36. The absorbent product of any of the claims 28 to 35, wherein the distance of the stiffened element to the longitudinal channel is greater than the distance of the stiffened element to any of the transverse and longitudinal edges adjacent the stiffened element.

## Patentansprüche

1. Absorbierendes Produkt (30), das dazu eingerichtet ist, von einer Frau in der Unterwäsche zur Damenhygiene getragen zu werden, mit einer flüssigkeitsdurchlässigen zum Körper weisenden Lage (2), einer flüssigkeitsundurchlässigen zur Kleidung weisendem Lage (4) und einem absorbierenden Element dazwischen, wobei das absorbierende Produkt (6) Folgendes aufweist:
einander gegenüberliegende longitudinale Ränder (8, 10), welche die Abmessung einer Breite festlegen, eine mittleren longitudinalen Achse (11) parallel zu den longitudinalen Rändern (8, 10), einander gegenüberliegende transversale Ränder (12, 14), welche die Abmessung einer Länge festlegen;
einen mittleren Bereich (18) mit einem ersten und einem zweiten distalen Ende (19, 20), die einander gegenüberliegen und eine Länge definieren, die ausreicht, um die Labia majora einer Frau im Gebrauch zu bedecken;
einen ersten Endbereich (22), der sich vom distalen Ende (19) des mittleren Bereich (18) erstreckt und dafür eingerichtet ist, wenigstens einen Teil des Mons pubis einer Frau im Gebrauch zu bedecken,
einen zweiten Endbereich (24), der sich von dem zweiten distalen Ende (29) des mittleren Bereichs (18) erstreckt und dafür eingerichtet ist, wenigstens einen Teil des posterior Perineum einer Frau im Gebrauch zu bedecken; **dadurch gekennzeichnet, daß** das absorbierende Produkt weiterhin Folgendes umfaßt:
wenigstens eine Anpassungseinrichtung (31, 32), die in wenigstens einem Endbereich (22, 24) enthalten ist, und die einen nicht versteiften Bereich (27, 29) aufweist, der an dem distalen Ende (19, 20) des mittleren Bereichs (18) angrenzt und an ein versteiftes Element (26, 28) angrenzt, wobei das versteifte Element (26, 28) innerhalb der transversalen und longitudinalen Ränder (12, 14; 8, 10) des absorbierenden Produkts (6) angeordnet ist, und das versteifte Element (26, 28) eine laterale Komponente (64, 65) aufweist, die sich transversal über den wenigstens einen Endbereich (22, 24) senkrecht zu der mittleren longitudinalen Achse (72) des absorbierenden Produkts erstreckt und mittig wenigstens 50% der Breite des absorbierenden Produkts (5) einnimmt;
wobei das versteifte Element (26, 28) weiterhin wenigstens eine senkrechte Komponente (66, 67, 68, 69) umfaßt, die sich longitudinal über wenigstens einen Teil eines Endbereichs erstreckt, wobei sich die senkrechte Komponente longitudinal über wenigstens einen Teil der lateralen Komponente erstreckt;
wobei sich das absorbierende Produkt bevorzugt in dem nicht versteiften Bereich (27, 29) umbiegt und das versteifte Element (26, 28) sich einem transversalen Umbiegen widersetzt.

2. Absorbierendes Produkt nach Anspruch 1, bei dem die Anpassungseinrichtung (31, 32) wenigstens eine Achse senkrecht zu der mittleren longitudinalen Achse aufweist und der Widerstand zum Umbiegen des absorbierenden Elements (6) entlang der wenigstens einen Achse im Vergleich nicht größer ist als ohne das versteifte Element (26, 28) in dem absorbierenden Element (6) der Anpassungseinrichtung (31, 32).

3. Absorbierendes Produkt nach Anspruch 1 oder 2, bei dem das absorbierende Produkt eine Damenbinde, eine Slipeinlage oder ein Inkontinenzprodukt ist.

4. Absorbierendes Produkt nach einem der vorhergehenden Ansprüche, bei dem das versteifte Element (26, 28) außerdem wenigstens eines der folgenden Merkmale aufweist: einen eingeprägten Kanal (32), einen verdichteten Bereich, eine Hervorstehung über die zum Körper weisende Lage (2), einen zusätzlichen Materialstreifen, der wenigstens die zum Körper weisende Lage (2) verstärkt, einen Bereich der absorbierenden Struktur (7), der Harz enthält, einen Bereich der absorbierenden Struktur, der einen Schmelzkleber enthält, oder Kombinationen davon.

5. Absorbierendes Produkt nach einem der vorhergehenden Ansprüche, bei dem das versteifte Element mittig weniger als 95% der transversalen Breite des Endbereichs einnimmt.

6. Absorbierendes Produkt nach Anspruch 5, bei dem ein mittiger äußerer Rand von 5% der transversalen Breite der Endbereiche frei von der Besetzung mit dem versteiften Element ist.

7. Absorbierendes Produkt nach Anspruch 4, bei dem das versteifte Element wenigstens einen eingeprägten Kanal (38) aufweist, der in die absorbierende Struktur eingedrückt ist, insbesondere bei dem der eingeprägte Kanal (38) nach unten in die zum Körper weisende durchlässige Lage (2) und in die absorbierenden Struktur (7) darunter eingedrückt ist.

8. Absorbierendes Produkt nach einem der vorhergehenden Ansprüche, bei dem der eingeprägte Kanal nach oben in die zur Kleidung weisende Lage und in die absorbierende Struktur darüber eingedrückt ist.

9. Absorbierendes Produkt nach einem der vorhergehenden Ansprüche, bei dem er eingeprägte Kanal (36) nach unten in die zum Körper weisende Lage (2), in die absorbierende Struktur (6) und in die zur Kleidung weisende Lage(4) darunter eingedrückt ist.

10. Absorbierendes Produkt nach einem der vorhergehenden Ansprüche, bei dem der eingeprägte Kanal (38) über die zur Kleidung weisende Lage (4) hervorragt.

11. Absorbierendes Produkt nach einem der vorhergehenden Ansprüche, bei dem der eingeprägte Kanal (34) nach oben in die zur Kleidung weisenden Lage (4), in die absorbierende Struktur (7) und in die zum Körper weisende Lage (2) darüber eingedrückt ist.

12. Absorbierendes Produkt nach Anspruch 11, bei dem der eingeprägte Kanal (42) über die zur Kleidung weisende Lage (2) hervorsteht.

13. Absorbierendes Produkt nach einem der vorhergehenden Ansprüche mit wenigstens einem eingeprägten Kanal (48), der nach unten in die zur Kleidung weisende Lage (2) und in die absorbierende Struktur (6) darunter eingedrückt ist, und mit wenigstens einem eingeprägten Kanal (50), der nach oben in die zur Kleidung weisende Lage (45) und in die absorbierende Struktur (6) darüber eingedrückt ist.

14. Absorbierendes Produkt nach einem der vorhergehenden Ansprüche, bei dem die Abmessung der Länge etwa in dem Bereich zwischen 101,6 und 330,2 mm (4 und 13 Zoll) liegt und die Abmessung der Breite etwa in dem Bereich zwischen 38,1 und 101,6 mm (1. 5 und 4 -Zoll) liegt.

15. Absorbierendes Produkt nach einem der vorhergehenden Ansprüche, bei dem das versteifte Element etwa zwischen 3,175 mm (0,125 Zoll) und 63,5 mm (2,5 Zoll) von den Ränder des Endbereichs nach innen versetzt angeordnet ist.

16. Absorbierendes Produkt nach einem der vorhergehenden Ansprüche, bei dem das versteifte Element (54, 56) eine laterale Komponente, die transversal über dem Endbereich (58, 62) angeordnet ist, und zusätzlich wenigstens eine senkrechte Komponente (66, 67, 68, 69) umfaßt, die orthogonal zu der lateralen Komponente (64, 65) und parallel zu der mittleren longitudinalen Achse (72) des absorbierenden Produkt (80) liegt und näher an dem longitudinalen Rand (74, 76) als an der mittleren longitudinalen Achse (72) liegt.

17. Absorbierendes Produkt nach einem der vorhergehenden Ansprüche, bei dem das versteifte Element aus kontinuierlichen Segmenten, diskontinuierlichen Segmenten, graden Segmenten, gekrümmten Segmenten oder einer Kombination davon gebildet ist.

18. Absorbierendes Produkt nach einem der vorhergehenden Ansprüche, welches eine rechteckige Form in der ebenen Ansicht aufweist und bei dem die longitudinalen Ränder parallel und äquidistant zueinander sind.

19. Absorbierendes Produkt nach einem der vorhergehenden Ansprüche, bei dem wenigstens ein Teil eines mittleren Bereichs eine Breite aufweist, die geringer ist als die transversale Breite von wenigstens einem der Endbereiche.

20. Absorbierendes Produkt nach einem der vorhergehenden Ansprüche, das zusätzlich wenigstens einen Streifen zum Anbringen an die Kleidung aufweist, welcher an einer der longitudinalen Ränder des absorbierenden Elements angebracht ist.

21. Absorbierendes Produkt nach einem der vorhergehenden Ansprüche, das weiterhin wenigstens einen Streifen zum Anbringen an die Kleidung aufweist, der von einem der longitudinalen Ränder des absorbierenden Elements nach innen versetzt angebracht ist.

22. Absorbierendes Produkt nach einem der vorhergehenden Ansprüche, das zusätzlich wenigstens einen Bund (136, 137) aufweist, der an einem der longitudinalen Ränder (140, 141) des absorbierenden Elements (160) angebracht ist.

23. Absorbierendes Produkt nach einem der vorhergehenden Ansprüche, das zusätzlich wenigstens einen Bund (136, 137) aufweist, der an einem der longitudinalen Ränder (140, 141) des absorbierenden Elements (160) angebracht ist, und wenigstens einen Streifen (161, 162) zum Anbringen an die Kleidung aufweist, der von einem der longitudinalen Ränder (165, 166) des absorbierenden Elements (135) nach innen versetzt angebracht ist.

24. Absorbierendes Produkt nach einem der vorhergehenden Ansprüche, bei dem die absorbierende Struktur im mittleren Bereich (152) dicker als in den Endbereichen (146, 147) ist.

25. Absorbierendes Produkt nach einem der vorhergehenden Ansprüche, bei dem die absorbierende Struktur einen absorbierenden Kern umfaßt.

26. Absorbierendes Produkt nach einem der vorhergehenden Ansprüche, bei dem die absorbierende Struktur eine Übertragungslage und einen absorbierenden Kern umfaßt.

27. Absorbierendes Produkt nach Anspruch 25 oder Anspruch 26, bei dem das Material, welches den absorbierenden Kern umfaßt, aus einer Gruppe ausgewählt ist, welche aus Holzfaserstoff, Gewebe, synthetischen Fasern, die behandelt sind, um sie hydrophil zu machen, hydrophobe synthetische Fasern in Gemischen mit hydrophilen Fasern, Torfmoos, superabsorbierende Partikel, superabsorbierende Fasern und aus Kombinationen davon besteht.

28. Absorbierendes Produkt nach Anspruch 1, bei dem das Produkt wenigstens eine versteifende Einrichtung in dem mittleren Bereich von dem versteiften Element beabstandet aufweist und bei dem die versteifende Einrichtung der Eigenschaft des absorbierenden Produkts dient, sich einem longitudinalen Umbiegen zu widersetzen.

29. Absorbierendes Produkt nach Anspruch 28, bei dem die versteifende Einrichtung wenigstens eines der folgenden Merkmale aufweist: einen eingeprägten Kanal, eine zusätzliche Dicke von absorbierendem Material, einen starren Einsatz, einen halbstarren Einsatz oder Kombinationen davon.

30. Absorbierendes Produkt nach Anspruch 28 oder Anspruch 29, bei dem der eingeprägte Kanal ein longitudinaler Kanal ist, der sich zwischen der mittleren longitudinalen Achse und dem longitudinalen Rand befindet.

31. Absorbierendes Produkt nach Anspruch 30, bei dem der longitudinale Kanal gerade ist.

32. Absorbierendes Produkt nach Anspruch 30, bei dem der longitudinale Kanal gekrümmt ist, wobei er sich nach innen zu der longitudinalen Achse krümmt.

33. Absorbierendes Produkt nach Anspruch 32, bei dem der longitudinale Kanal gekrümmt ist, wobei er sich nach innen zu der longitudinalen Achse krümmt.

34. Absorbierendes Produkt nach Anspruch 29, bei dem der halbstarre Einsatz Torfmoos enthält.

35. Absorbierendes Produkt nach Anspruch 29, bei dem der halbstarre Einsatz eine Plastikbahn ist.

36. Absorbierendes Produkt nach einem der Ansprüche 28 bis 35, bei dem der Abstand zwischen dem versteiften Element und dem longitudinalen Kanal größer ist als der Abstand zwischen dem versteiften Element und einem beliebigen der transversalen oder longitudinalen Ränder, die an dem versteiften Element angrenzen.

## Revendications

1. Produit absorbant (30) adapté pour être porté dans un sous-vêtement par une femme pour l'hygiène féminine comprenant une couche faisant face au corps perméable aux liquides (2), une couche faisant face au vêtement imperméable aux liquides (4), un élément absorbant entre celles-ci, le produit absorbant (6) ayant :
des bords longitudinaux (8, 10) se faisant face l'un à l'autre définissant une dimension de largeur, un axe longitudinal central (11) parallèle aux bords longitudinaux (8, 10), des bords transversaux (12, 14) se faisant face l'un à l'autre définissant une dimension de longueur ;
une région centrale (18) ayant une première et une seconde extrémités distales (19, 20) se faisant face l'une à l'autre définissant une longueur qui est suffisante pour couvrir les grandes lèvres d'une femme lors de l'utilisation ;
une première région d'extrémité (22) s'étendant depuis la première extrémité distale (19) de la région centrale (18) et étant adaptée pour couvrir au moins une portion du mont Vénus d'une femme lors de l'utilisation ;
une seconde région d'extrémité (24) s'étendant depuis la seconde extrémité distale (29) de la région centrale (18) et étant adaptée pour couvrir au moins une portion du périnée postérieur d'une femme lors de l'utilisation ;et **caractérisé en ce que** le produit absorbant comprend en outre
au moins un moyen de conformation (31, 32) contenu dans au moins une région d'extrémité (22, 24), comprenant une région non rigide (27, 29) adjacente à l'extrémité distale (19, 20) de la région centrale (18) et adjacente à un élément rigide (26, 28), l'élément rigide (26, 28) étant situé vers l'intérieur des bords transversaux et longitudinaux (12, 14 ; 8, 10) du produit absorbant (6), l'élément rigide (26, 28) ayant un composant latéral (64, 65) s'étendant de manière transversale à travers au moins une région d'extrémité (22, 24) perpendiculairement à l'axe longitudinal central (72) du produit absorbant (6) et occupant centralement au moins 50 % de la largeur du produit absorbant (6) ;
l'élément rigide (26, 28) comprenant en outre au moins un composant perpendiculaire (66, 67, 68, 69) s'étendant longitudinalement à travers au moins une portion de la région d'extrémité, le composant perpendiculaire s'étendant longitudinalement au-delà d'au moins une portion du composant latéral ;
dans lequel le produit absorbant se plie de préférence dans la région qui n'est pas rigide (27, 29) et l'élément rigide (26, 28) résiste à la torsion transversale.

2. Produit absorbant selon la revendication 1, dans lequel le moyen de conformation (31, 32) contient au moins un axe, perpendiculaire à l'axe longitudinal central, et la résistance au pliage de l'élément absorbant (6) le long du au moins un axe n'est pas supérieure à celle qu'on observe en l'absence de l'élément rigide (26, 28) dans l'élément absorbant (6) du moyen de conformation (31, 32).

3. Produit absorbant selon la revendication 1 ou 2, dans lequel le produit absorbant est une serviette hygiénique, un protège-slip ou un produit pour l'incontinence.

4. Produit absorbant selon l'une quelconque des revendications précédentes, dans lequel l'élément rigide (26, 28) comprend en outre au moins un élément parmi un canal gaufré (32), une région densifiée, une projection au-dessus de la couche faisant face au corps (2), une bande de matière supplémentaire renforçant au moins la couche faisant face au corps (2), une région contenant de la résine de la structure absorbante (7), une région contenant un produit thermoplastique de la structure absorbante, ou une combinaison de ceux-ci.

5. Produit absorbant selon l'une quelconque des revendications précédentes, dans lequel l'élément rigide occupe centralement moins de 95 % de la largeur transversale de la région d'extrémité.

6. Produit absorbant selon la revendication 5, dans lequel un rebord extérieur centré de 5 % de la largueur transversale des régions d'extrémité est libre de l'occupation de l'élément rigide.

7. Produit absorbant selon la revendication 4, dans lequel l'élément rigide comprend au moins un canal gaufré (38) imprimé dans la structure absorbante, en particulier dans lequel le canal gaufré (38) est imprimé vers le bas dans la couche perméable faisant face au corps (2) et la structure absorbante (7) en dessous.

8. Produit absorbant selon l'une quelconque des revendications précédentes, dans lequel le canal gaufré est imprimé vers le haut dans la couche faisant face au vêtement et la structure absorbante au-dessus.

9. Produit absorbant selon l'une quelconque des revendications précédentes, dans lequel le canal gaufré (36) est imprimé vers le bas dans la couche faisant face au corps (2), la structure absorbante (6) et la couche faisant face au vêtement (4) en dessous.

10. Produit absorbant selon l'une quelconque des revendications précédentes, dans lequel le canal gaufré (38) fait saillie au-delà de la couche faisant face au vêtement (4).

11. Produit absorbant selon l'une quelconque des revendications précédentes, dans lequel le canal gaufré (34) est imprimé vers le haut dans la couche faisant face au vêtement (4), la structure absorbante (7) et la couche faisant face au corps (2) au-dessus.

12. Produit absorbant selon la revendication 11, dans lequel le canal gaufré (42) fait saillie au-delà de la couche faisant face au corps (2).

13. Produit absorbant selon l'une quelconque des revendications précédentes, ayant au moins un canal gaufré (48) imprimé vers le bas dans la couche faisant face au corps (2) et la structure absorbante (6) en dessous et au moins un canal gaufré (50) imprimé vers le haut dans la couche faisant face au vêtement (45) et la structure absorbante (6) au-dessus.

14. Produit absorbant selon l'une quelconque des revendications précédentes, dans lequel la dimension de longueur est environ dans la plage comprise entre 101,6 et 330,2 mm (4 et 13 pouces), la dimension de largeur est environ dans la plage comprise entre 38,1 et 101,6 mm (1,5 et 4 pouces ).

15. Produit absorbant selon l'une quelconque des revendications précédentes, dans lequel l'élément rigide est situé environ entre 3,175 mm (0,125 pouce) et 63,5 mm (2,5 pouces) vers l'intérieur des bords de la région d'extrémité.

16. Produit absorbant selon l'une quelconque des revendications précédentes, dans lequel l'élément rigide (54, 56) comprend un composant latéral (64, 65) situé de manière transversale à travers la région d'extrémité (58, 62) et en outre au moins un composant perpendiculaire (66, 67, 68, 69) orthogonal au composant latéral (64, 65) et parallèle à l'axe longitudinal central (72) du produit absorbant (80), et est plus proche du bord longitudinal (74, 76) que de l'axe longitudinal central (72).

17. Produit absorbant selon l'une quelconque des revendications précédentes, dans lequel l'élément rigide est constitué de segments continus, de segments discontinus, de segments droits, de segments courbes ou d'une combinaison de ceux-ci.

18. Produit absorbant selon l'une quelconque des revendications précédentes, ayant une forme rectangulaire en vue en plan, et dans lequel les bords longitudinaux sont parallèles les uns aux autres et équidistants les uns des autres.

19. Produit absorbant selon l'une quelconque des revendications précédentes, dans lequel au moins une partie de la région centrale a une largeur inférieure à la largeur transversale d'au moins une des régions d'extrémité.

20. Produit absorbant selon l'une quelconque des revendications précédentes, ayant en outre au moins un onglet de fixation au vêtement fixé à un des bords longitudinaux de l'élément absorbant.

21. Produit absorbant selon l'une quelconque des revendications précédentes, ayant en outre au moins un onglet de fixation au vêtement fixé vers l'intérieur de l'un des bords longitudinaux de l'élément absorbant.

22. Produit absorbant selon l'une quelconque des revendications précédentes, ayant en outre au moins un revers (136, 137) attaché à un des bords longitudinaux (140, 141) de l'élément absorbant (160).

23. Produit absorbant selon l'une quelconque des revendications précédentes, ayant en outre au moins un revers (136, 137) attaché à un des bords longitudinaux (140, 141) de l'élément absorbant (160) et au moins un onglet de fixation au vêtement (161, 162) fixé vers l'intérieur d'un des bords longitudinaux (165, 166) de l'élément absorbant (135).

24. Produit absorbant selon l'une quelconque des revendications précédentes, dans lequel la structure absorbante est plus épaisse dans la région centrale (152) que dans les régions d'extrémité (146, 147).

25. Produit absorbant selon l'une quelconque des revendications précédentes, dans lequel la structure absorbante est constituée d'un coeur absorbant.

26. Produit absorbant selon l'une quelconque des revendications précédentes, dans lequel la structure absorbante est constituée d'une couche de transfert et d'un coeur absorbant.

27. Produit absorbant selon la revendication 25 ou la revendication 26, dans lequel la matière constituant le noyau absorbant est sélectionnée parmi le groupe constitué de la pâte de bois, du tissu, de fibres synthétiques traitées pour les rendre hydrophiles, de fibres synthétiques hydrophobes mélangées à des fibres hydrophiles, de mousse de sphagnum, de particules super absorbantes, de fibres super absorbantes et d'une combinaison de ceux-ci.

28. Produit absorbant selon la revendication 1, dans lequel le produit comprend au moins un moyen rigidifiant dans la région centrale éloignée de l'élément rigide et dans lequel le moyen rigidifiant facilite la capacité du produit absorbant à résister au pliage longitudinal.

29. Produit absorbant selon la revendication 28, dans lequel le moyen rigidifiant comprend au moins un élément parmi un canal gaufré, une épaisseur supplémentaire de matière absorbant, un insert rigide, un insert semi-rigide ou des combinaisons de ceux-ci.

30. Produit absorbant selon la revendication 28 ou la revendication 29, dans lequel le canal gaufré est un canal longitudinal situé entre l'axe longitudinal central et le bord longitudinal.

31. Le produit absorbant selon la revendication 30, dans lequel le canal longitudinal est droit.

32. Produit absorbant selon la revendication 30, dans lequel le canal longitudinal est courbe, se courbant vers l'intérieur vers l'axe longitudinal.

33. Produit absorbant selon la revendication 32, dans lequel le canal longitudinal est courbe, se courbant vers l'intérieur vers l'axe longitudinal.

34. Produit absorbant selon la revendication 29, dans lequel l'insert semi-rigide contient de la mousse de sphagnum.

35. Produit absorbant selon la revendication 29, dans lequel l'insert semi-rigide est une feuille de plastique.

36. Produit absorbant selon l'une quelconque des revendications 28 à 35, dans lequel la distance de l'élément rigide au canal longitudinal est supérieure à la distance de l'élément rigide à l'un quelconque des bords transversaux et longitudinaux adjacents à l'élément rigide.
